# EUROPEAN PATENT APPLICATION

(11) **EP 4 450 017 A1**
(43) Date of publication of application: **23.10.2024**
(21) Application number: 23168373.1
(22) Date of filing: 18.04.2023
(51) Int. Cl.: A61B 90/20, A61B 90/00, A61B 90/50, A61F 9/007, A61B 17/00, H04N 19/00

(54) **APPARATUS FOR AN OPTICAL IMAGING SYSTEM, OPTICAL IMAGING SYSTEM, METHODS AND COMPUTER PROGRAM**

(71) Applicant: Leica Instruments (Singapore) Pte. Ltd., Singapore 608924 (SG)
(72) Inventor: YANG, Gao, 608924 Singapore (SG)
(74) Representative: 2SPL Patentanwälte PartG mbB

(57) **Abstract**

Examples relate to an apparatus for an optical imaging system. The apparatus comprises one or more processors and one or more storage devices. The apparatus is configured to receive sensor data of a sensor of the optical imaging system. The sensor data is received from the optical imaging system. The sensor data is indicative of a live view of the sample through microscope of the optical imaging system. Further, the apparatus is configured to determine predictive data indicative of a predicted view of the sample. The predicted data is determined based on the sensor data. Further, the apparatus is configured to determine auxiliary data indicative of auxiliary information for a user of the optical imaging system. The auxiliary data is determined based on the predictive data. The apparatus is further configured to transmit an auxiliary signal indicative of auxiliary data.

## Description

### Technical field

Examples relate to an apparatus for an optical imaging system, such as a surgical optical imaging system, an optical imaging system, methods, and a computer program.

### Background

In surgical optical imaging system, a surgeon often uses a main pair of oculars or ocular displays to perform a surgical procedure. A live view of a sample under test may be displayed to the surgeon using the ocular or ocular display. For some operations the live view of the sample may be unsatisfactory for performing an operation. The surgeon may rely on auxiliary information providing guidance and/or assistance for the operation. For example, for cataract surgery an artificial lens implant needs to be arranged in a certain position of the eye of the patient. The eye of the patient may be non-static, such that auxiliary information needs to be updated to a current status of the eye. The need for updating the auxiliary information may lead to a delay of the auxiliary information compared to the live view, leading to a reduced performance of the optical imaging system.

Thus, there may be a desire for an improved concept for a for low-latency auxiliary information presentation.

### Summary

This desire is addressed by the subject-matter of the independent claims.

The concept proposed in the present disclosure is based on the insight, that a time delay between a live view and auxiliary information, e.g., caused by video processing design, can be reduced by calculating the auxiliary information for the future. For example, the auxiliary information may be rendered ahead based on the current live view by predicting an image of the live view in the future with the time delay. In this way, the auxiliary information can be provided substantially simultaneously with the live view. Thus, a latency between the live view and the auxiliary information can be reduced or even omitted.

Examples provide an apparatus for an optical imaging system. The apparatus comprises one or more processors and one or more storage devices. The apparatus is configured to receive sensor data of a sensor of the optical imaging system. The sensor data is received from the optical imaging system. The sensor data is indicative of a live view of the sample through microscope of the optical imaging system. Further, the apparatus is configured to determine predictive data indicative of a predicted view of the sample. The predicted data is determined based on the sensor data. Further, the apparatus is configured to determine auxiliary data indicative of auxiliary information for a user of the optical imaging system. The auxiliary data is determined based on the predictive data. The apparatus is further configured to transmit an auxiliary signal indicative of the auxiliary data. By generating the predicted data indicative of the predicted view of the sample auxiliary information can be provided for a future state of the sample. In this way, latency of the auxiliary information can be reduced. The predicted data may be related to an actual delay of a video processing design. Thus, displaying the auxiliary information can be matched with displaying the live view. Therefore, a misalignment between the live view and auxiliary information can be reduced or even avoided. The predicted data may be a view of the sample of at a future state of the sample. For example, the predicted data may be indicative of the state of the sample in the near future, e.g., in a time corresponding to a time needed for video processing design, a time for processing the life image to generate the visual overlay with the auxiliary information and/or a time for generating the auxiliary information. For example, the predicted data may be indicative of a future state of the sample, in a future corresponding to a time delay between live view and displaying the auxiliary information. The auxiliary information may be information provided to the user to guide and/or assist the user, e.g., a surgeon during an operation. For example, the auxiliary information may provide the user details about an orientation and/or a position of a structure, e.g., an artificial lens implant, used during an operation. For example, the auxiliary information can provide a user details about a region of interest, e.g., for removing the cataract without damaging other parts of thy of the patient. The auxiliary signal may be transmitted to a display device or a storage device, such like a frame buffer. The live view may be transmitted to a display device or a storage device, such like a frame buffer. For example, the live view and the auxiliary signal may be displayed on a display device by use of a visual overlay.

In an example, the apparatus may be further configured to determine the predicted data based on data extrapolation. Data extrapolation refers to the process of estimating values or trends outside the range of observed data based on a mathematical model or pattern that is observed in the available data. Data extrapolation may allow to make predictions or estimates about future values based on the observed patterns or trends in the available data. In this way, a future state of the sample can be determined based on the live view. Thus, the sensor data can be utilized to generate the predicted data in a facilitated way.

In an example, the apparatus may be further configured to determine the predicted data based on a change detection algorithm. For example, a sequence of past and/or present inputs to a data analyzer may be used for the change detection algorithm. The change detection algorithm may be advantageously to detect sudden or unexpected changes of the sample.

In an example, the apparatus may be further configured to obtain setting data of the optical imaging system. The setting data may be indicative of a setting of the optical imaging system. Further the apparatus may be configured to determine the predicted data based on the setting data. For example, the setting of the optical imaging system may define a time delay for video processing design, a delay of a sensor. Thus, the setting of the optical imaging system may be indicative of the time delay between the live view and the auxiliary information. In this way, the apparatus can determine a time in the future for which the future state of the sample has to be determined to reduce the latency of the auxiliary information.

In an example, the apparatus may be further configured to determine a required prediction time for the prediction data based on the setting data. The required prediction time may be the time delay between displaying the live view and displaying the auxiliary information. For example, the prediction time may be the time in the future for which the state of the sample corresponds to time needed to display the auxiliary information. Thus, the generation of the auxiliary information can be increased since a time in the future for which the auxiliary information may be needed can be determined in an improved way.

In an example, the apparatus may be further configured to determine a required prediction time for the prediction data based on the sensor data. For example, the sensor data may be indicative of a time of acquiring an image. Thus, the apparatus can determine a time delay based on sensor data, e.g., a timestamp of sensor data, in a facilitated way.

In an example, the apparatus may be further configured to determine confidence data indicative of a reliability of the predicted data. The confidence data can be used to inform the user about the reliability of the predicted data. For example, if the reliability of the predicted data is low then the reliability of the auxiliary information may also be low. The user can be informed about the low reliability of the auxiliary information, such that the user can take certain measures, e.g., pause the operation, turn off the auxiliary information, use auxiliary information with higher latency.

In an example, the apparatus may be further configured to stop the transmission of the auxiliary signal if the reliability of the predicted data is below a threshold. In this way, it can be ensured that the auxiliary information is only displayed to the user if the user can actually use the auxiliary information.

In an example, the apparatus may be further configured to determine the auxiliary data further indicative of a reliability of the auxiliary information. The auxiliary data may be determined based on the confidence data. In this way, the user can be easily informed about the reliability of the auxiliary information.

In an example, the sensor data may be further indicative of processed sensor data of the sensor. For example, the sensor data may be processed before the predicted data is determined. In this way, the prediction model can be separated from the processing of the sensor data. Thus, different entities may be assigned with the computational tasks belonging to processing of the sensor data or the prediction model. This may improve a design of the optical imaging system.

Examples provide an optical imaging system comprising an apparatus as described above.

In an example, the optical imaging system may further comprise a mixer device. The mixer device may be configured to receive sensor data of a sensor of the optical imaging system. The sensor data is indicative of a live view of the sample through microscope of the optical imaging system. Further, the mixer device may be configured to receive auxiliary data, e.g., from the apparatus. Further, the mixer device may be configured to generate a visual overlay comprising a visual representation of the live view and the auxiliary information. The visual overlay may be generated based on the sensor data and the auxiliary data. Thus, the mixer device can generate an output signal comprising the live view and the auxiliary information to compensate for the time delay for determining/generating the auxiliary information.

Examples provide a method for an optical imaging system comprising receiving sensor data of a sensor of the optical imaging system from the optical imaging system. The sensor data is indicative of a live view of a sample through a microscope of the optical imaging system. Further, the method comprises determining predicted data indicative of a predicted view of the sample. The predicted data is determined based on the sensor data. Further, the method comprises determining auxiliary data indicative of auxiliary information for a user of the optical imaging system and transmitting an auxiliary signal indicative of the auxiliary data. The auxiliary data is determined based on the predicted data.

Examples provide a method for an optical imaging system. The method comprises receiving sensor data of a sensor of the optical imaging system, the sensor data indicative of a live view of a sample through a microscope of the optical imaging system. The sensor data may be received from a sensor of the optical imaging system and/or may from a storage device of the optical imaging system. The method may further comprise receiving auxiliary data, e.g., from the apparatus tasked with determining the auxiliary information and generating, based on the sensor data and the auxiliary data, a visual overlay comprising a visual representation of the live view and the auxiliary information.

Various examples of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
- Figs. 1a and 1b: show schematic diagrams of examples of an apparatus for an optical imaging system and of a corresponding optical imaging system;
- Figs. 2a-2c: show different examples of flow diagrams of systems for generating the visual overlay comprising a visual presentation of a live view and auxiliary information;
- Figs. 3a-3c: show different examples of the insight generation block of Fig. 2.
- Fig. 4: shows a flow chart of an example of a method for an optical imaging system that uses reflectance imaging;
- Fig. 5: shows a flow chart of another example of a method for an optical imaging system; and
- Fig. 6: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

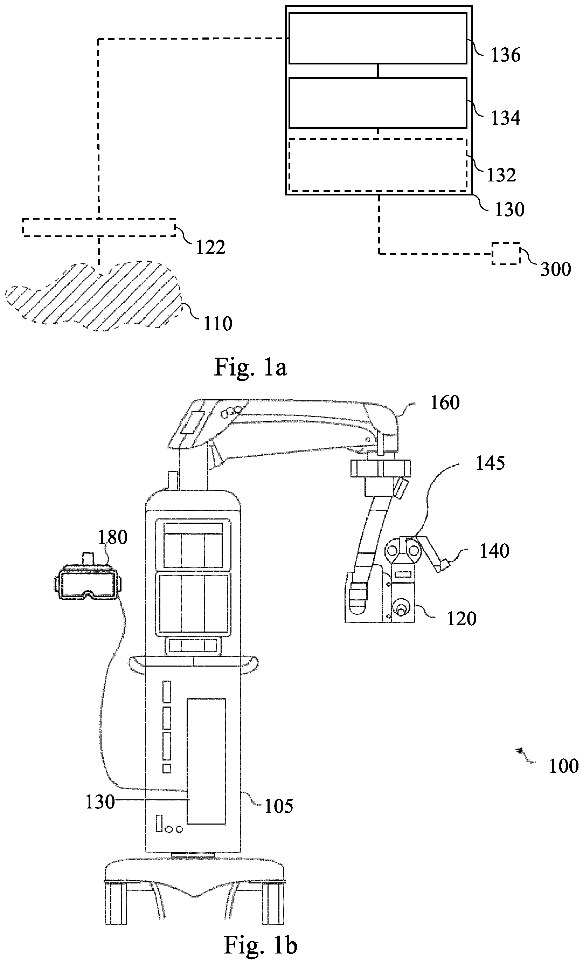

Figs. 1a and 1b show schematic diagrams of an example of an apparatus 130 for an optical imaging system 100 and of a corresponding optical imaging system 100 comprising the apparatus 130. The apparatus 130 is tasked with controlling various aspects of a microscope 120 of the optical imaging system 100, which may be a surgical optical imaging system, and of the entire optical imaging system 100 and/or with processing various types of sensor data of the optical imaging system 100. Consequently, the apparatus 130 may be implemented as a computer system, which interfaces with the various components of the optical imaging system, e.g., the sensor 122.

The apparatus 130 comprises, as shown in Fig. 1a, one or more processors 134 and one or more storage devices 136. Optionally, the apparatus 130 further comprises one or more interfaces 132. The one or more processors 134 are coupled to the one or more storage devices 136 and to the optional one or more interfaces 132. In general, the functionality of the apparatus 130 may be provided by the one or more processors 134 (for determining the predicted data and/or the auxiliary data), in conjunction with the one or more interfaces 132 (for exchanging information, e.g., with the sensor 122 or a display device/storage device, e.g., to transmit the auxiliary signal to the display device/storage device) and/or with the one or more storage devices 136 (for storing and/or retrieving information).

The apparatus 130 is configured to receive sensor data of the sensor 122 of the optical imaging system 100. The sensor data is received from the optical imaging system 100. The sensor data is indicative of a live view of the sample 110 through microscope 120 of the optical imaging system 100. For example, the sensor data is received from the sensor 122. Alternatively, the sensor data may be received from a storage device of the optical imaging system 100. The live view refers to a real-time display of the sample 110. For example, a live view may allow the user to see a continuous and up-to-date display of the sample 110 being acquired by the microscope 120.

The apparatus 130 is further configured to determine predicted data indicative of a predicted view of the sample. The predicted data is determined based on the sensor data, e.g., by data extrapolation and/or change detection algorithm as described below. The predicted view of the sample 110 may be indicative of a state of the sample in the future. During an operation the sample 110 may be non-static. For example, an orientation of the sample may change during the operation. By determining the predicted data state of the sample 110 in the future a likely state of the sample 110 can be predicted.

As described above, a user of the optical imaging system 100 may rely on auxiliary information for performing a task, such like an operation. Since the generation and/or determination of the auxiliary information needs a certain time, a time delay may exist between live view of the sample 110 and auxiliary information associated with the live view of the sample 110. For example, a whole frame of the life image may be needed to be stored and processed to generate the auxiliary information. Thus, at least two data transfer blocks may be needed for transmitting the auxiliary information. In contrast, if the live view is stored in a storage device, such like a frame buffer, only one data transfer block may be needed for transmission. The different number of needed data transfer blocks results in a time delay between live view and auxiliary information. This time delay could cause overall long latency in visual information for information integration of the auxiliary information. Alternatively, the live view path (for transmitting the live view) could be prioritized to have minimal latency, while auxiliary information experience longer latency and may be presented later than the live view. However, this may decrease a performance for the user.

The apparatus 130 can be used to maintain responsiveness of the live view, e.g., a life surgical video. For this the apparatus 130 may be further configured to determine auxiliary data indicative of auxiliary information for a user of the optical imaging system 100. The auxiliary data is determined based on the predicted data. In this way, the auxiliary information can be adapted to an expected time delay. A computerization of an expected time delay between live view and auxiliary information can be compensated by determining the auxiliary information based on the predicted data. For example, the auxiliary information can be determined for a future state of the sample 110 in the future defined by the time delay between live view and auxiliary information. In this way, the auxiliary information can be determined for the predicted live view. Further, the auxiliary information for the predicted live view can be displayed with an actual live view, which may result in displayed auxiliary information associated with the actual live view with reduced or without any latency.

Thus, the apparatus 130 can still prioritize the live view for low latency by providing auxiliary information based on a predicted live view. For example, to compensate for the expected time delay in the auxiliary information path (e.g., caused by video processing design, determination of the auxiliary information), the apparatus 130, e.g., an auxiliary information processor, can predict the live view and presents a future state of the sample 110. The prediction may be based on data extrapolation and/or a change detection algorithm. So when the auxiliary information is combined (e.g., by a mixer, editor) with the live view of the sample 110, there is less or even no time difference (and such latency) between them.

To combine the auxiliary information the apparatus 130 is further configured to transmit an auxiliary signal indicative of the auxiliary data. The auxiliary signal can be transmitted to a video mixer device, video editor device, storage device and/or a display device. For example, the video mixer device may mix the live view with the auxiliary data to generate a visual overlay of the sample 110 with the auxiliary information. This visual overlay may be displayed on a display device. In this way, the auxiliary information can be provided to the user of the optical imaging system 100 with reduced latency.

The proposed concept is built around two main components - the microscope 120, which comprises the optical components, and which may house the display device 300 being used to view the sample 110, and the apparatus 130, which is used to control the optical imaging system 100, process sensor data of the microscope 120, e.g., the sensor 122, and to generate an auxiliary signal.

In general, a microscope, such as the microscope 120, is an optical instrument that is suitable for examining objects that are too small to be examined by the human eye (alone). For example, a microscope 120 may provide an optical magnification of a sample, such as a sample 110 shown in Fig. 1a. In modern microscopes, the optical magnification is often provided for a camera or an imaging sensor, such as the optical imaging sensors 122 of the microscope 120. The microscope 120 may further comprise one or more optical magnification components that are used to magnify a view of the sample 110, such as an objective.

There are a variety of different types of optical imaging systems. If the optical imaging system 100 is used in the medical or biological fields, the sample 110 may be a sample of organic tissue, e.g., arranged within a petri dish or present in a part of a body of a patient. In some examples of the present disclosure, e.g., as shown in Fig. 1b, the optical imaging system 100 may be a surgical optical imaging system, e.g., an optical imaging system that is to be used during a surgical procedure, such as an oncological surgical procedure or during tumor surgery. However, the proposed concept may also be applied to other types of microscopy, e.g., microscopy in a laboratory or microscopy for the purpose of material inspection.

As is evident, the optical imaging system 100 comprises a number of components, such as the apparatus 130, the microscope 120 with the at least one optical imaging sensors 122, an optional main pair of ocular displays 140, an optional secondary pair of ocular displays 145 and a head-mounted display 180. In the configuration shown in Fig. 1a, the microscope 120 may be a non-stereoscopic microscope 120 that only supports a two-dimensional view of the sample 110 using the sensor 122. However, other implementations may be possible, such as an implementation with a single pair of optical imaging sensors for visible light imaging, or a pair of optical imaging sensors for visible light and fluorescence imaging, or four image sensors for both visible light (reflectance) imaging and fluorescence imaging, two optical imaging sensors for providing the two stereo channels for visible light imaging and two optical imaging sensors for providing the two stereo channels for fluorescence imaging.

In an example, the apparatus 130 may be further configured to determine the predicted data based on data extrapolation. The data extrapolation can be based on a single frame or multiple frames of the live view. The data extrapolation may involve projecting trends or patterns observed in a single/multiple frame/s to estimate values or trends in future frames. For example, if a particular pixel in an image is observed to be changing at a certain rate over time, it may be possible to extrapolate this trend to estimate the pixel's value in future frames.

If the sensor data comprises a time stamp a set of time series can determined based on the sensor data. Alternatively, it is possible to use image recognition algorithms to extract the time information from the live view. Once the time stamp is extracted, the time stamp could be used to generate a set of times by extrapolating from the known time to future times. For example, if the time stamp on the image is x, then it may be possible to use data extrapolation to generate a set of times at regular intervals beyond the known time x, such as every 16.67 µs in a 60 frames-per-second video system, for example. In this way, predicted data for a future state of the sample 110 can be generated in a facilitated way.

In an example, the apparatus 130 may be further configured to determine the predicted data based on a change detection algorithm. For example, previous frames, e.g., at least two frames, of the live view may be used to analyze an inherence change in the live view. A change detection algorithm is a type of algorithm used in image processing that is designed to identify changes in the content of images or videos over time. The basic idea behind change detection algorithms is to compare the content of two or more images or video frames captured at different times and to identify any differences or changes between them. For example, pixel-based change detection, feature-based change detection and/or model-based change detection can be used.

The prediction model may be based on data extrapolation and/or change detection algorithm. Additionally, the prediction model may depend on a use case, e.g., a domain of the surgery. Additionally, the prediction model can be based on machine learning algorithm. The live view can be used as input data and a user input can be used as ground truth.

In an example, the apparatus 130 may be further configured to obtain setting data of the optical imaging system. The setting data may be indicative of a setting of the optical imaging system 100. Further the apparatus 130 may be configured to determine the predicted data based on the setting data. For example, a time delay between the live view and the auxiliary information may be defined by a setting of the optical imaging system 100. A processing circuitry of the optical imaging system 100 may be tasked with video processing. The video processing of the live view, e.g., to generate the auxiliary information, may need a certain time, which results in a time delay of the auxiliary information in comparison to the live view. By obtaining setting data the apparatus 130 can determine the time delay, such that the predicted data can be determined based on the time delay. In this way, the predicted data can be indicative of a future state of the sample 110 needed to compensate for the time delay between live view and auxiliary information. Thus, the auxiliary information can be determined based on the future state of the sample 110 with respect to an actual time delay of the optical imaging system 100. In this way, auxiliary information can be provided to reduce or avoid latency between live view and auxiliary information in a facilitated way.

The setting data may be obtained by receiving from a storage device (e.g., of the optical imaging system 100) information about a setting of the optical imaging system 100 and/or by determining a setting of the optical imaging system 100, e.g., a used sensor, a used video mixer device, a used rendering device, for example.

In an example, the apparatus 130 may be further configured to determine a required prediction time for the prediction data based on the setting data. The required prediction time may correspond to the time delay between displaying the live view and displaying the auxiliary information. The value of the required prediction time may be identical to the value of the time delay. For example, the required prediction time may define a future state of the sample in the future with a time interval equal to the value of the time delay. For example, the live view of the sample 110 can be displayed nearly instantaneously on a display device since no additional image processing may be required. In contrast, the auxiliary information needs to be generated, such that it fits to the live view of the sample 110. Therefore, a time delay to generate the auxiliary information cannot be avoided.

The required prediction time may be a time needed to generate the auxiliary information. For example, the required prediction time may be associated with a future state of the sample 110, such that the auxiliary information can be generated for the future state of the sample 110 to compensate the time delay for generating the auxiliary information.

In an example, the apparatus 130 may be further configured to determine a required prediction time for the prediction data based on the sensor data. The apparatus 130 can be configured to determine the time delay using the timestamp on the live view, e.g., on each frame of the live view. Based on the timestamp of a frame of the live view, a time delay caused by the generation of the auxiliary information can be determined. In this way, the time delay can be determined in a facilitated way.

In an example, the apparatus 130 may be further configured to determine confidence data indicative of a reliability of the predicted data. The confidence data may indicate how good the prediction of the future state of the sample 110 is. For example, the confidence data can be used determine whether the auxiliary information is displayed to the user or not. If the confidence data indicates low reliability of the predicted data also the auxiliary information may have a low reliability. In this case the auxiliary information may be not transmitted to avoid displaying auxiliary information with low reliability. Alternatively, the confidence data indicates high reliability of the predicted data information to the user may be displayed that the auxiliary information is of high reliability. In this way, an acceptance of the user can be increased.

In an example, the apparatus 130 may be further configured to stop the transmission of the auxiliary signal if the reliability of the predicted data is below a threshold. For example, the value for the reliability of the auxiliary information (also referred to as confidence score) may be from 0 (lowest reliability) to 10 (highest reliability). If the confidence score is from 0 to 3 no auxiliary signal may be transmitted by the apparatus 130. If the value is at least 4 the apparatus 130 may transmit the auxiliary signal.

In an example, the apparatus 130 may be further configured to determine the auxiliary data further indicative of a reliability of the auxiliary information. The auxiliary data may be determined based on the confidence data. In this way, an information about the reliability of the auxiliary information can be displayed to the user. Because the reliability of the auxiliary information can be low, e.g., caused by the predicted data not fully accurate, the auxiliary information may include the confidence score. For example, the confidence can be visualized using color, e.g., green or yellow icon, transparency of the auxiliary information, and/or textual notation to inform the user of such uncertainties. The confidence score may be determined by the apparatus 130 based on the confidence data.

For example, if the confidence score of the auxiliary information is from 4 to 7, the user can be informed about the middle reliability, e.g., by a yellow icon. For example, if the value of the reliability of the auxiliary information is from 8 to 10, the user can be informed about the high reliability, e.g., by a green icon. In this way, the user can be informed in a facilitated way about a reliability of the auxiliary information. Thus, a user experience can be increased.

Additionally, the auxiliary information can also be selectable. For example, if the reliability of the auxiliary information is too low for a user, the user can turn off the auxiliary information. For example, the user can receive information about the reliability of the auxiliary information and can decide based on the reliability whether he wants to use the auxiliary information or not. Thus, when accuracy outweighs perceived fluidity, the user can turn off the generation of the auxiliary information by the apparatus 130. In this case, the auxiliary data can be generated with a higher latency and increased accuracy. For example, the apparatus 130 may receive a trigger signal indicative of an input of the user to stop the determination of the auxiliary data. The trigger signal can be received from an input device, a storage device, for example.

In an example, the sensor data may be further indicative of processed sensor data of the sensor. For example, the sensor data may be processed before the predicted data is determined. In this way, the prediction model can be separated from the processing of the sensor data. Thus, different entities, such like processing circuitries, may be assigned with the computational tasks belonging to processing of the sensor data or the prediction model.

Fig. 1b shows a schematic diagram of an example of a surgical optical imaging system 100 comprising the microscope 120 and the apparatus 130. In general, a (surgical) optical imaging system is a system that comprises a microscope 120 and additional components, which are operated together with the microscope 120. In other words, a (surgical) optical imaging system is a system that comprises the microscope 120 and one or more additional components, such as the apparatus 130 (which may be a computer system being adapted to control and, for example, generate the control signal), an illumination system (which is used to illuminate a sample being imaged by the microscope 120), additional sensors, displays etc.

The surgical optical imaging system 100 shown in Fig. 1b comprises a number of optional components, such as a base unit 105 (which may comprise the apparatus 130) with a stand, ocular displays 140, 145 that are arranged at the microscope 120, a head-mounted display 180, and a (robotic or manual) arm 160 which holds the microscope 120 in place, and which is coupled to the base unit 105 and to the microscope 120. In general, these optional and non-optional components may be coupled to the apparatus 130, which may be configured to control and/or interact with the respective components.

In an example, the optical imaging system 100 may further comprise a mixer device. The mixer device may be configured to receive sensor data of a sensor 122 of the optical imaging system. The sensor data is indicative of a live view of the sample 110 through microscope 120 of the optical imaging system 100. Further, the mixer device may be configured to receive auxiliary data, e.g., from the apparatus 130. Further, the mixer device may be configured to generate a visual overlay comprising a visual representation of the live view and the auxiliary information. The visual overlay may be generated based on the sensor data and the auxiliary data. Thus, the mixer device can generate an output signal comprising the live view and the auxiliary information. The output signal can be transmitted from the mixer device to a display device 300 or a storage device, such like a frame buffer. In this way, the mixer device can trigger displaying the live view and the auxiliary information on the display device 300, for example. Since the visual overlay is based on the auxiliary data, the visual overlay may comprise a compensation of a time delay to determine/generate the auxiliary information.

As shown in Fig. 1a the optional one or more interfaces 132 is coupled to the respective one or more processors 134 at the apparatus 130. In examples the one or more processors 134 may be implemented using one or more processing units, one or more processing devices, any means for processing, such as a processor, a computer or a programmable hardware component being operable with accordingly adapted software. Similar, the described functions of the one or more processors 134 may as well be implemented in software, which is then executed on one or more programmable hardware components. Such hardware components may comprise a general-purpose processor, a Digital Signal Processor (DSP), a micro-controller, etc. The one or more processors 134 is capable of controlling the one or more interfaces 132, so that any data transfer that occurs over the one or more interfaces 132 and/or any interaction in which the one or more interfaces 132 may be involved may be controlled by the one or more processors 134.

In an embodiment the apparatus 130 may comprise a memory, e.g., the one or more storage devices 136 and at least one or more processors 134 operably coupled to the memory and configured to perform the method described below.

In examples the one or more interfaces 132 may correspond to any means for obtaining, receiving, transmitting or providing analog or digital signals or information, e.g., any connector, contact, pin, register, input port, output port, conductor, lane, etc. which allows providing or obtaining a signal or information. The one or more interfaces 132 may be wireless or wireline and it may be configured to communicate, e.g., transmit or receive signals, information with further internal or external components.

The apparatus 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system. The apparatus 130 may be part of the optical imaging system 100. Alternatively the apparatus 130 may be external to the optical imaging system 100, e.g., may be part of the display device 300.

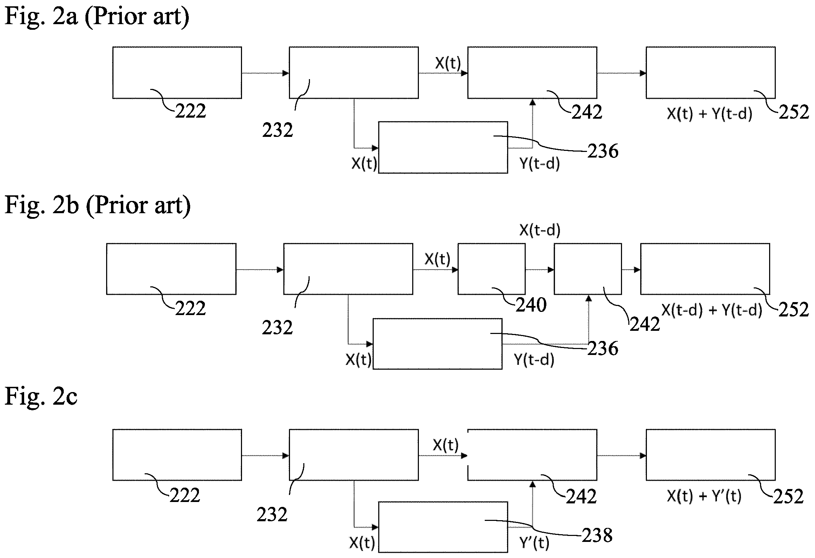

More details and aspects are mentioned in connection with the examples described below. The example shown in Fig. 1 may comprise one or more optional additional features corresponding to one or more aspects mentioned in connection with the proposed concept or one or more examples described below (e.g., Fig. 2 - 6).

Figs. 2a-2c show different examples of flow diagrams of systems for generating the visual overlay comprising a visual presentation of a live view and auxiliary information. Figs. 2a and 2b show two different examples of prior art systems. Fig. 2a shows the system without compensation of the time delay between auxiliary information and live view. The live view is prioritized over the auxiliary information in Fig. 2a. In 222 a sensor may acquire the image data of a sample, such like a live view of the sample. In 232 the live view of the sample is processed, e.g., by video processing. For example, buffering of the live view may be performed to reduce a faltering live view display. The video processing in 232 may require a time X(t), e.g., to store a whole frame. Thus, a time delay of X(t) may be added by the video processing in 232. Thus, the live view of the sample may be received at a mixer device after a time X(t).

In 236 the auxiliary information may be generated in an insight generation block. The generation of the auxiliary information may require an additional time Y(t-d). Thus, the auxiliary information may be received at the mixer device after a time Y(t-d).

The insight generation block may take video data X, such like a processed live view, at time t, denoted as X(t). The insight generation block may generate auxiliary information such as surgical guidance graphics Y. Because there is time needed to transfer the data in and out, as well as processing the information to get Y from X, there is a total delay of d. Therefore, without compensation, when X(t) is presented at the input of the insight generation block, Y(t-d), which is auxiliary information aligned with time t-d, is available at its output. As a result, X(t) is mixed with a delayed overlay, Y(t-d) in 242. The mixer device may mix the (processed) live view and the auxiliary information in 242. For example, the mixer device may put one pixel of the auxiliary information on top of one pixel of the live view.

In 252 X(t) (the processed live view) may be displayed with the delayed Y(t-d) (auxiliary information).

Alternatively, as shown in Fig. 2b the live view can be delayed in 240 to match the insight generation of the auxiliary information. However, the end result is both live view and auxiliary are displayed delayed in 252.

Fig. 2c shows an example of a flow diagram according to the invention. In Fig. 2c a compensation of the time delay caused by the generation of the auxiliary information is performed. The compensation of the time delay is performed in 238. With compensation, a temporally extrapolated and/or predicted auxiliary information Y'(t) may be output from the insight generation block in 238. For example, the extrapolated and/or predicted auxiliary information may be determined based on the predicted data comprising a future state of a sample, as described with reference to Fig. 1. The extrapolated and/or predicted auxiliary information may be determined by the apparatus as described with reference to Fig. 1.

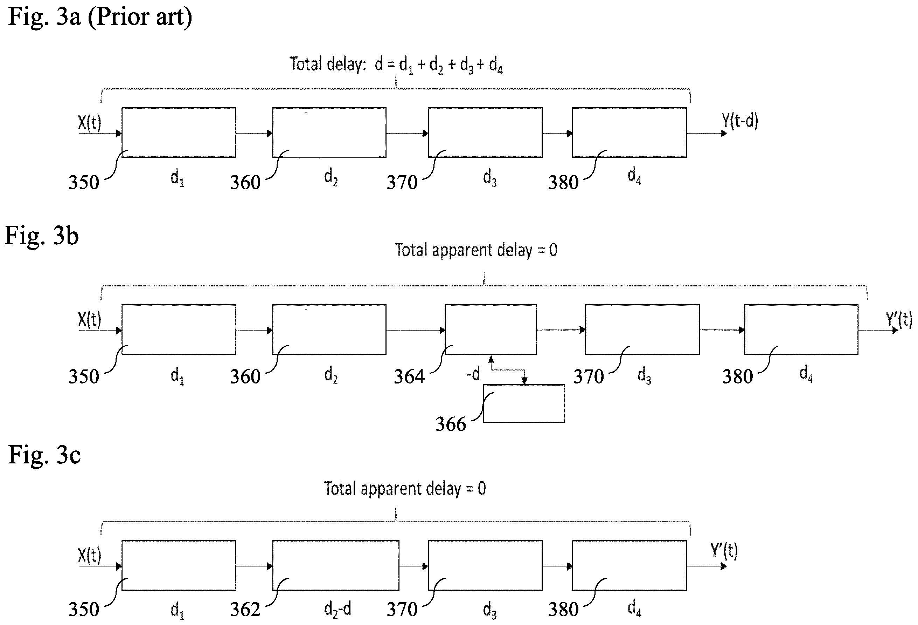

The extrapolated and/or predicted auxiliary information can be aligned in time with the processed live view X(t). In this way, the time delay of the auxiliary information can be compensated. Thus, the user of the imaging system can receive the auxiliary information substantially simultaneously with the live view. A more detailed description of the insight generation block 238 is given in Fig. 3.

More details and aspects are mentioned in connection with the examples described above and/or below. The example shown in Fig. 2 may comprise one or more optional additional features corresponding to one or more aspects mentioned in connection with the proposed concept or one or more examples described above (e.g., Fig. 1) and/or below (e.g., Fig. 3 -6).

Figs. 3a-3c show different examples of the insight generation block of Fig. 2. Fig. 3a shows an example of a prior art insight generation block without compensation. Without compensation, the total time delay d can be thought of as the total of data transfer delays d₁, d₄ (e.g., in 350, 380), processing delay d₂ (in 360), and rendering delay d₃ (in 370); d = d₁ + d₂ + d₃ +d₄. Therefore, the display auxiliary information is a delayed overlay Y(t-d).

To compensate for the total time delay a prediction of the future state of the sample can be performed. Thus, the auxiliary information can be determined based on the prediction of the future state of the sample allowing to introduce a negative time delay -d. In Figs. 3b and 3c a prediction to reduce the total time delay, especially to 0 is performed.

As can be seen in Fig. 3b in 364 a prediction to determine prediction data can be performed. The prediction may predict a future state of the sample. The future state may be a state of the sample in the future corresponding to the time delay between the live view and the auxiliary information. Thus, the prediction at 364 may generate a negative time delay of -d (according to the time delay between the live view and the auxiliary information). In this way, the total time delay may be 0. For performing the prediction in 364 an access to storage device may be necessary. For example, a separate prediction circuitry, such like an auxiliary information processor, may be connected to the output of the information processing circuitry to perform block 364. To store previous states and take in the latest state to predict a future state d time ahead the storage device may be accessed in 366. In this way, a temporally extrapolated and/or predicted auxiliary information Y'(t) can be generated.

Alternatively, in Fig. 3c the prediction can be performed in the same block as the processing of the sensor data in 362. For example, the information processing block may contain a storage device for previous states and the auxiliary information processor internally. The auxiliary information processor may comprise the storage device for storing the previous states. In this way, a temporally extrapolated and/or predicted auxiliary information Y'(t) can be generated.

For example, in cataract surgeries where an artificial lens implant, such like a toric lens, is used to correct astigmatism, the rotational orientation of the toric lens needs to be aligned with a pre-determined angle of the patient's eye. As the eye can move and rotate during surgery, an image analysis module can be used to track such motion and draw a compass over the live video to indicate the correct angle in real time. Because the analysis usually takes place outside of the video camera, the output rendering is expected to fall behind the live video, by d frames (the total time delay).

By modelling the motion of the eye using its x, y positions and/or its rotational angle and/or their temporal derivatives, prediction data can be determined indicative of the position and angle of the eye d frames in the future. The output of the position and angle d frames in the future may allow to determine the extrapolated and/or predicted auxiliary information Y'(t). Using the extrapolated and/or predicted auxiliary information Y'(t) the d-frame delay can be canceled, and the auxiliary information can be aligned in an improved way with the live view.

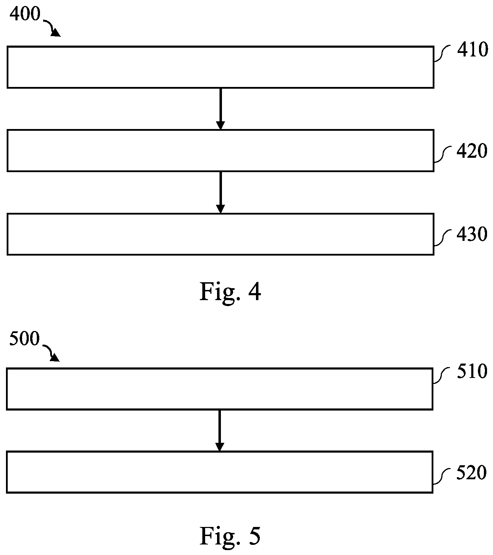

More details and aspects are mentioned in connection with the examples described above and/or below. The example shown in Fig. 3 may comprise one or more optional additional features corresponding to one or more aspects mentioned in connection with the proposed concept or one or more examples described above (e.g., Fig. 1-2) and/or below (e.g., Fig. 4 - 6).

Fig. 4 shows a flow chart of an example of a method 400. The method 400 is for an optical imaging system, e.g., the optical imaging system described with reference to Fig. 1b. The method 400 may be performed by an apparatus as described with reference to Fig. 1a.

The method 400 comprises receiving 410 sensor data of a sensor of the optical imaging system from the optical imaging system. The sensor data is indicative of a live view of a sample through a microscope of the optical imaging system. Further, the method 400 comprises determining 420 predicted data indicative of a predicted view of the sample. The predicted data is determined 420 based on the sensor data. Further, the method 400 comprises determining 430 auxiliary data indicative of auxiliary information for a user of the optical imaging system and transmitting an auxiliary signal indicative of the auxiliary data. The auxiliary data is determined 430 based on the predicted data.

More details and aspects are mentioned in connection with the examples described above and/or below. The example shown in Fig. 4 may comprise one or more optional additional features corresponding to one or more aspects mentioned in connection with the proposed concept or one or more examples described above (e.g., Fig. 1-3) and/or below (e.g., Fig. 5 - 6).

Fig. 5 shows a flow chart of another example of a method 500. The method 500 is for an optical imaging system, e.g., the optical imaging system described with reference to Fig. 1b. The method 500 may be performed by an apparatus of the optical imaging system, e.g., a video mixer device.

The method 500 comprises receiving 510 sensor data of a sensor of the optical imaging system, the sensor data indicative of a live view of a sample through a microscope of the optical imaging system. The sensor data may be received from a sensor of the optical imaging system and/or may from a storage device of the optical imaging system.

The method 500 may further comprise receiving 520 auxiliary data, e.g., from the apparatus tasked with determining the auxiliary information (e.g., as described with reference to Fig. 1a) and generating 530, based on the sensor data and the auxiliary data, a visual overlay comprising a visual representation of the live view and the auxiliary information.

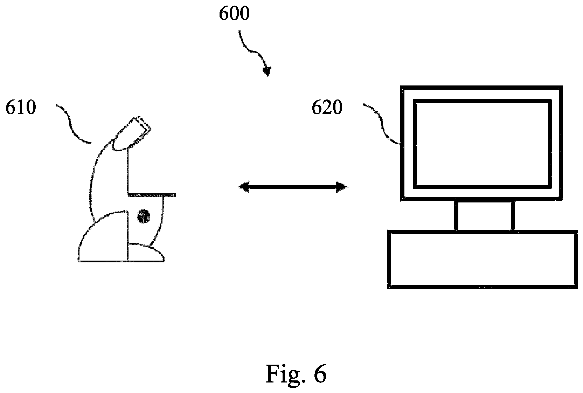

More details and aspects are mentioned in connection with the examples described above and/or below. The example shown in Fig. 5 may comprise one or more optional additional features corresponding to one or more aspects mentioned in connection with the proposed concept or one or more examples described above (e.g., Fig. 1 - 4) and/or below (e.g., Fig. 6).

Some embodiments relate to a microscope comprising an apparatus as described in connection with one or more of the Figs. 1 to 5. Alternatively, a microscope may comprise of or can be communicatively connected to an apparatus as described in connection with Fig. 1. Fig. XXX shows a schematic illustration of a system 600, e.g., an optical imaging system, configured to perform a method described herein, e.g., with reference to Figs. 4 or 5. The system 600 comprises a microscope 610 and a computer system 620. The microscope may comprise the apparatus as described above, e.g., with reference to Fig. 1. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 620 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

More details and aspects are mentioned in connection with the examples described above. The example shown in Fig. 6 may comprise one or more optional additional features corresponding to one or more aspects mentioned in connection with the proposed concept or one or more examples described above (e.g., Fig. 1 - 5).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

If some aspects have been described in relation to a device or system, these aspects should also be understood as a description of the corresponding method and vice versa. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 optical imaging system
105 base
110 sample
120 microscope
122 sensor
130 apparatus
132 interface
134 processor
136 storage device
140, 145 ocular display
160 arm
180 head-mounted display
222 image acquisition
232 video processing
236 determination of auxiliary information
242 mixing
252 displaying
350 data transfer
360 information processing
362 information processing and prediction
364 prediction
370 rendering
380 data transfer
400 method
410 receiving sensor data
420 determining predicted data
430 determining auxiliary data
440 transmitting an auxiliary signal
500 method
510 receiving sensor data
520 receiving auxiliary data
530 generating a visual overlay
600 system
610 microscope
620 computer system

## Claims

1. An apparatus (130) for an optical imaging system (100), comprising one or more processors (134) and one or more storage devices (136), wherein the apparatus (130) is configured to:
receive, from the optical imaging system (100), sensor data of a sensor (122) of the optical imaging system (100), the sensor data indicative of a live view of a sample (110) through a microscope (122) of the optical imaging system (100);
determine, based on the sensor data, predicted data indicative of a predicted view of the sample;
determine, based on the predicted data, auxiliary data indicative of auxiliary information for a user of the optical imaging system;
transmit an auxiliary signal indicative of the auxiliary data.

2. The apparatus (130) according to claim 1, wherein
the apparatus (130) is further configured to determine the predicted data based on data extrapolation.

3. The apparatus (130) according to any one of the preceding claims, wherein
the apparatus (130) is further configured to determine the predicted data based on a change detection algorithm.

4. The apparatus (130) according to any one of the preceding claims, wherein
the apparatus (130) is further configured to:
obtain setting data of the optical imaging system, the setting data indicative of a setting of the optical imaging system; and
determine the predicted data based on the setting data.

5. The apparatus (130) according to claim 4, wherein
the apparatus (130) is further configured to determine a required prediction time for the prediction data based on the setting data.

6. The apparatus (130) according to any one of the preceding claims, wherein
the apparatus (130) is further configured to determine a required prediction time for the prediction data based on the sensor data.

7. The apparatus (130) according to any one of the preceding claims, wherein
the apparatus (130) is further configured to determine confidence data indicative of a reliability of the predicted data.

8. The apparatus (130) according to claim 7, wherein
the apparatus (130) is further configured to stop the transmission of the auxiliary signal if the reliability of the predicted data is below a threshold.

9. The apparatus (130) according to claim 7 or 8, wherein
the apparatus (130) is further configured to determine, based on the confidence data, the auxiliary data further indicative of a reliability of the auxiliary information.

10. The apparatus (130) according to any one of the preceding claims, wherein
the sensor data is further indicative of processed sensor data of the sensor.

11. An optical imaging system (100), comprising
an apparatus (130) according to any one of the preceding claims.

12. The optical imaging system (100) according to claim 11, further comprising
a mixer device configured to:
receive sensor data of a sensor (122) of the optical imaging system (100), the sensor data indicative of a live view of a sample (110) through a microscope (120) of the optical imaging system (100);
receive auxiliary data; and
generate, based on the sensor data and the auxiliary data, a visual overlay comprising a visual representation of the live view and the auxiliary information.

13. A method (400) for an optical imaging system, comprising:
receiving (410), from the optical imaging system, sensor data of a sensor of the optical imaging system, the sensor data indicative of a live view of a sample through a microscope of the optical imaging system;
determining (420), based on the sensor data, predicted data indicative of a predicted view of the sample;
determining (430), based on the predicted data, auxiliary data indicative of auxiliary information for a user of the optical imaging system; and
transmitting (440) an auxiliary signal indicative of the auxiliary data.

14. A method (500) for an optical imaging system, comprising:
receiving (510) sensor data of a sensor of the optical imaging system, the sensor data indicative of a live view of a sample through a microscope of the optical imaging system;
receiving (520) auxiliary data; and
generating (530), based on the sensor data and the auxiliary data, a visual overlay comprising a visual representation of the live view and the auxiliary information.

15. A computer program with a program code for performing the method according to claim 13 or 14 when the computer program is executed on a processor.
